# EUROPÄISCHE PATENTSCHRIFT

(11) **EP 0 571 422 B1**
(45) Veröffentlichungstag und Bekanntmachung des Hinweises auf die Patenterteilung: **31.08.1994**
(21) Anmeldenummer: 92903988.1
(22) Anmeldetag: 12.02.1992
(51) Int. Cl.: A61B 17/12

(54) **IMPLANTAT FÜR ORGANWEGE IN SPIRALFORM**
SPIRAL IMPLANT FOR BODILY DUCTS
IMPLANT EN SPIRALE POUR CONDUITS DU CORPS

(30) Priorität: 15.02.1991 DE 4104702
(43) Veröffentlichungstag der Anmeldung: 01.12.1993
(73) Patentinhaber: NEUSS, Malte, D-78464 Konstanz (DE)
(72) Erfinder: NEUSS, Malte, D-78464 Konstanz (DE)
(74) Vertreter: Sternagel, Hans-Günther, Dr.
(86) Internationale Anmeldenummer: EP9200294
(87) Internationale Veröffentlichungsnummer: WO9214408

(56) Entgegenhaltungen:
- DD-A- 223 065

## Beschreibung

Gegenstand der Erfindung sind Implantate für Organwege, vorzugsweise für Blutgefäße, deren Sekundärform entweder aus einer Primärdrahtspirale aus Metall oder einem Primärrohr aus Kunststoff durch Verwinden oder Wickeln ausgebildet wird, wobei die Sekundärform zum Einführen gestreckt und mit dem Plazieren im Organ wieder ausgebildet wird.

Seit mehr als 10 Jahren werden in der interventionellen Radiologie Metallspiralen zum Gefäßverschluß mittels Kathetern implantiert. Derartige Spiralen sind in der Literatur wiederholt beschrieben. W. Castaneda-Zuniga et al. beschreiben in Radiology 136;230-231 (Juli 1980) eine verbesserte Form der sogenannten Gianturco-Spiralen, die mittels Kathetern aus Polyurethan oder Polyethylen eingeführt werden können. Die Primärspirale wird durch geeignete Techniken zu einem Knäuel oder einer Sekundärspirale verformt. Um die Spirale in ihrer Sekundärform implantieren zu können, wird diese durch Aufziehen der Primärspirale auf einen Führungsdraht gestreckt. Beim Implantieren durch einen Katheter auf dem Führungsdraht wird auf dem Führungsdraht noch eine weitere verschiebbare Spirale angeordnet, die dazu dient, das Implantat, d.h. die Primärspirale aus dem Katheter zu schieben und vom Führungsdraht abzuschieben, so daß sich die Sekundärform zurückbildet und die Sekundärspirale oder das Knäuel im zu verschließendan Gefäß bleibt.

J. Anderson, S. Walace und C. Gianturco beschreiben in Am. J. Roentgenol 129: 795-798 (November 1977) die Vorzüge der Replazierbarkeit der Spirale vor dem endgültigen Abwurf. Ihre schraubenförmige Korkenzieherverbindung hat aber den Nachteil, daß durch das scharfe Ende der Spirale die Gefahr der Gefäßperforation besteht. Außerdem kann die Spirale durch Drehung beim Dekonnektieren deplaziert werden. Dafür sprechen auch die zahlreichen Komplikationen bei den Tierversuchen.

M.J. Mazer und Mitarbeiter beschreiben in Radiology 138; 37-46 (Januar 1981) die Nachteile der bekannten Technik und weisen auf Fehlschläge durch falsche Auswahl der Größe der Sekundärspirale und auf Risiken ungeeigneter Abstreifer hin.

In DD-A-223 065 ist eine Vorrichtung zum Verschließen von Adern beschrieben, die aus einem spitzenpräparierten Herz- oder Angiographie-Katheter, einem Schubelement (Pusher), einem Verschlußkörper und aus einem Kerndraht besteht, an dessen vorderem Ende der Verschlußkörper aufgeschoben und ablösbar befestigt ist. Der Kerndraht ist im Innern des Schubelementes bewegbar geführt und mit dem aufgeschobenen Verschlußkörper und dem Schubelement derart über die Kathetermündung hinaus weiter in die Ader einführbar, daß der Verschlußkörper auf dem Kerndraht verbleibt und durch diesen auch außerhalb des Katheters geführt ist. Durch Festhalten des Kerndrahtes und weiteres Verschieben des Schubelementes ist der Verschlußkörper vom Kerndraht abstreifbar. Dies kann durch Zurückziehen des Kerndrahtes in das Schubelement geschehen. Der Verschlußkörper ist über seine gesamte Länge und über seinen gesamten Umfang mit einem textilen Fasermaterial versehen ist. Er weist an seinem hinteren Ende einen hülsenförmigen Teil auf, in den der Kerndraht derartig einführbar ist, daß der Verschlußkörper mit Hilfe des Schubelementes vom Kerndraht einwandfrei ablösbar ist.

Aus DD-A-158 084 ist ein Verschlußkörper geeignet zum intraarteriellen und intrayenösen Verschluß von Blutgefäßen bekannt, bei dem eine Drahtspirale unter Anwendung höherer Umformungsgrade so zu einem Schraubenfederkörper ausgebildet wird, daß er unter Anwendung eines äußeren Zwanges zu einer geraden Drahtspirale elastisch umformbar ist und nach teilweisem Wegfall desselben eine knotenförmige Gestalt annimmt. Aufgabe der vorliegenden Erfindung ist es, die konstruktive Gestaltung der Implantate und des Einführdrahtes zu verbessern, so daß eine Möglichkeit besteht, vor dem endgültigen Ablösen der Implantate von den zur Einführung benutzten Hilfsmitteln diese Implantate ggf. wieder zurückzuholen bzw. die Positionierung im Organweg zu verändern.

Diese Aufgabe wird gelöst durch ein Implantat für Organwege, das aus einer Primärspirale aus Metall oder einem Primärrohr aus alastischem Kunststoff gebildet wird, wobei das vordere Ende der Primärspirale oder des Primärrohres geschlossen ist und der hintere Bereich als Klemmsitz für einen Führungsdraht ausgebildet ist und das Implantat eine Sekundärform vergrößerten Außendurchmessers aufweist, die durch Aufschieben der Primärspirale oder des Primärrohres auf den Führungsdraht gestreckt werden kann und sich bei Herausziehen des Führungsdrahtes oder Abschieben vom Führungsdraht durch im Material vorhandene Rückstellkräfte zurückbildet, dadurch gekennzeichnet, daß in Abstand von 0,5 mm bis 2 mm vom hinteren Ende der Primärspirale oder des Primärrohres, deren/dessen Querschnitt auf einer Strecke von 0,01 mm bis 10 mm durch Verringerung des Innendurchmessers in mindestens einer radialen Richtung modifiziert ist.

Die Erfindung umfaßt weiterhin eine Kombination einer Vorrichtung zur Einführung dieser Implantate in einen Organweg mit Einführkatheter, Abstreifen und Führungsdraht für das Implantat, mit diesen Implantaten wobei die zum Verschieben der Primärspirale oder des Primärrohres umd zum Überwinden der Klemmkraft mit dem als Einführhilfe dienenden Führungsdraht erforderliche Kraft 0,5 N bis 10 N, vorzugsweise 1,5 N bis 3 N, beträgt.

Die Primärspiralen mit Durchmessern von 0,2 mm bis 3 mm, vorzugsweise 0,4 mm bis 1,5 mm werden aus Metalldraht mit Durchmessern von 0,06 mm bis 0,6 mm, vorzugsweise 0,1 mm bis 0,4 mm gewickelt und anschließend wird die sekundäre Form ausgebildet. Die Form kann aber auch direkt beim Wickeln der Primärspirale mitausgebildet werden. Geeignete Materialien für die Implantate mit einer Primärspirale aus Metall sind chirurgischer Stahldraht mit Federeigenschaften, rostender oder nicht rostender Federstahldraht, Federdraht aus unedelen Metallen, der mit korrosionsbeständigen Metallen wie Tantal, Titan, Platin, Gold oder Keramikwerkstoffen beschichtet ist. Der Draht kann runden, ovalen oder rechteckigen Querschnitt aufweisen.

Die Primärrohre für Implantate werden aus medizinisch verträglichen elastischen thermoplastischen Polymeren, wie Polyurethanhomo- oder Copolymeren, Polyolefincopolymeren, Siliconelastomeren hergestellt, Geeignet sind auch resorbierbare Polymere, wie aliphatische Polyester, z.B. Polydioxanon und anschließend wird die sekundäre Spiralform ausgebildet. Die Wandstärke der Primärrohre kann 0,08 mm bis 0,8 mm betragen, der Durchmesser der Primärrohre 0,3 mm bis 3 mm. Die Verengung des Querschnittes zum Erzielen eines Klemmsitzes im Falle von Primärrohren kann auch dadurch erfolgen, daß parallele Längsschlitze über den Umfang des Rohres eingeschnitten und Längsstreifen des Polymermaterials dauerhaft nach innen durch Eindrücken verformt werden. Der Vorteil dieser Gestaltung der Querschnittsverengung zum Erzeugen eines Klemmsitzes auf dem Führungsdraht besteht darin, daß die eingeformten längsverlaufenden Abschnitte eine vergrößerte Anlagefläche am Führungsdraht aufweisen.

Die Länge der Primärformen, d.h. der Primärspiralen oder Primärrohre kann 10 bis 500 mm, vorzugsweise 10 mm bis 200 mm betragen.

Kennzeichnend für die erfindungsgemäßen Implantate ist, daß die sekundäre Spiralform reversibel gestreckt werden kann durch Aufschieben der Primärspirale oder des Primärrohres auf einen Führungsdraht, dessen Innendurchmesser etwas geringer ist als der Innendurchmesser der Primärspirale oder des Primärrohres in den nicht modifizierten Teilen und die Sekundärform sich bei Herausziehen des Führungsdrahtes oder Abschieben vom Führungsdraht durch im Material vorhandene Rückstellkräfte zurückbildet. Ein solches elastisches Federverhalten und die daraus resultierenden Rückstellkräfte basieren auf den Eigenschaften der gewählten Materialien. Die Federwirkung kann beispielsweise durch Erwärmen des in die gewünschte Form gebrachten Materials und anschließendes rasches Abkühlen (Abschrecken) erreicht werden. Bei thermoplastischen Polymeren beruht deren Federwirkung häufig auf Ausbildung bestimmter Kristallstrukturen oder Einfrieren von Spannungen des Materials bei der Formgebung. Die Maßnahmen zur Einstellung des für die reversible Streckung erforderlichen Elastizitätsmoduls des Materials und die Auswahl der dafür geeigneten Materialien sind dem Fachmann bekannt und bereiten ihm keine Schwierigkeiten.

Durch das in der Nähe des hinteren Endes der Primärform der Implantate ausgebildete Teilstück mit modifiziertem Querschnitt wird ein Klemmsitz der Primärform, das ist annähernd die gestreckte Sekundärform auf dem Führungsdraht, erreicht, wobei die Klemmkraft jedoch nicht so groß ist, daß ein Verschieben der Primärform auf dem Führungsdraht und ein vollständiges Herausziehen des Führungsdrahtes bzw. Abstreifen vom Führungsdraht mittels eines Abstreifers nicht mehr möglich ist.

Die Plazierung des Implantats erfolgt mit einer Vorrichtung mit Einführkatheter, Abstreifer und Führungsdraht für das Implantat, wobei der Führungsdraht mit einer konischen Spitze versehen ist und unmittelbar nach der Spitze entweder eine Ringnut oder einen umlaufenden Wulst aufweist, um eine besonders hohe Klemmwirkung zwischen dem hinteren Bereich mit verringertem Innendurchmesser des Implantats und dem vorderen Bereich des Führungsdrahtes zu erreichen.

Zum Einführen in einen Organweg wird der Führungsdraht mit auf diesem aufgeschobenen Abstreifer und davor aufgeschobener Primärform des gestreckten Implantats in einen Katheter eingebracht. Der Durchmesser der Führungsdrähte kann bei massivem Querschnitt 0,07 bis 0,7 mm betragen. Die Replazierbarkeit des Implantats ist dadurch gegeben, daß die Haftreibung des Teilstückes mit modifiziertem, vorzugsweise verringertem Querschnitt auf dem Führungsdraht 2 bis 5 mal, vorzugsweise 2 bis 3 mal größer ist als die Kraft, die erforderlich ist, um die im Organweg durch teilweises Abstreifen vom Führungsdraht weitgehend vollständig ausgebildete Sekundärform durch Zurückziehen in den Einführkatheter wieder zu strecken. Die gewählte Obergrenze der Klemmkraft stellt sicher, daß bei unerwünschtem Verhaken des Implantats in der Wand des Organweges deren Beschädigung durch zu große Rückzugskraft vermieden wird. Der Klemmsitz der Primärform des Implantats auf dem Führungsdraht wird dadurch erreicht, daß der Querschnitt mindestens einer bis mehrerer Windungen der Primärspirale modifiziert ist durch Verringerung des Durchmessers der Primärspirale oder Ausbilden eines ovalen Querschnittes, dessen kleinerer Durchmesser kleiner ist als der Außendurchmesser des Führungsdrahtes. Die gewünschte Differenz zwischen Rückziehkraft in den Einführkatheter und der Kraft zur Überwindung des Klemmsitzes der Primärform auf dem Führungsdraht kann bei gegebenem modifizierten Querschnitt der Primärform auch durch Verwendung eines Führungsdrahtes mit reibungserhöhender Oberflächenausbildung im vorderen Bereich, beispielsweise aufgerauhter Oberfläche, einem umlaufenden Wulst oder mittels einer Ringnut im Führungsdraht, in die der modifizierte, vorzugsweise verengte Querschnitt zumindest teilweise einrastet, herbeigeführt werden.

Die oben beschriebene Vorrichtung ermöglicht die sichere Plazierung des erfindungsgemäßen Implantats in einem Organweg, wobei folgende Schritte durchlaufen werden:
a) Vorschieben des Katheters, in dessen Innenlumen sich das Implantat in gestrecktem Zustand auf dem Einführdraht befindet, innerhalb des Organweges in die Nähe der Stelle, wo das Implantat plaziert werden soll,
b) Verschieben des Einführdrahtes mit dem darauf in gestreckter Form befindlichen Implantat an die für die Plazierung vorgesehene Stelle,
c) Ausbilden der Sekundärform des Implantats durch Zurückziehen des Führungsdrahtes oder Vorschieben des Abstreifers,
d) weiteres Zurückziehen des Katheters und des Führungsdrahtes oder Vorschieben des Abstreifers bis nur noch die vordere Spitze des Führungsdrahtes im hinteren Bereich der Primärspirale oder dem Primärrohr im Klemmsitz gehalten wird,
e) Korrektur der Lage des Implantats bzw. falls notwendig Zurückziehen des Implantats in den Katheter,
f) Abstreifen des Implantats von dem Führungsdraht an der dafür vorgesehenen Stelle im Organweg mit Hilfe des Abstreifers.

Die Sekundärformen der erfindungsgemäßen Implantate können je nach der speziellen beabsichtigten Wirkung im Organweg unterschiedliche Formen aufweisen.

Als Prothese für einen Organweg ist eine zylindrische Form bevorzugt, wobei der Außendurchmesser des Zylinders mindestens so groß sein muß, wie der Innendurchmesser des zu stützenden Organweges, um eine sichere Plazierung zu gewährleisten. Um die Thrombosegefahr zu verringern, ist es bei dieser Verwendungsweise bevorzugt, die Oberfläche der Implantate zu heparinisieren. Im Falle von Implantaten aus Polymeren bereitet die chemische Bindung von Heparin an das Polymere keine Probleme und ist dem Fachmann bekannt. Im Falle von Implantaten aus Metall kann die Bindung des Heparins das Aufbringen einer dünnen haftenden Schicht eines zu chemischer Bindung befähigten medizinisch verträglichen Polymeren erfordern. Da die geeigneten Polymere, wie beispielsweise Polyvinylalkohol , Silicone oder Copolymere mit heparinbindenden Gruppen auf der Basis von Polyurethanen oder Polyolefinen filmbildende Eigenschaften aufweisen, können die Polymere entweder aus Dispersion, Emulsion oder Lösung in organischen Lösemitteln aufgebracht werden und der Film durch Abdampfen des flüssigen Mediums ausgebildet werden.

Um den offenen Innenquerschnitt derartiger Prothesen möglichst groß auszubilden, können die Primärspirale oder das Primärrohr des Implantats einen ovalen Querschnitt aufweisen und die zylindrische Sekundärform so ausgebildet werden, daß die Oberflächen mit dem größeren Krümmungsradius der Primärformen in der Sekundärform nebeneinander angeordnet werden und die Außenoberfläche der Sekundärform bilden.

Häufig ist jedoch eine gegenteilige Wirkung der Implantate erwünscht, Organwege sollen verengt oder durch das Implantat verschlossen werden. Um dies mit einer zylindrischen Sekundärform zu erreichen, kann entweder der Durchmesser entsprechend klein ausgebildet oder eine zylinderförmige Spirale gewickelt werden, bei der sich Windungen mit unterschiedlichem Durchmesser abwechseln, so daß der Innendurchmesser der Sekundärform in Längsachse der Spirale unterschiedlich ausgebildet ist, mit Einzelwindungen oder mehreren Windungen von minimal möglichem Krümmungsradius. Eine Verengung eines Organweges läßt sich auch durch einen Konus oder einen Doppelkonus mit größerem Durchmesser an den Enden der Sekundärform erreichen. Damit lassen sich Strömungen in Gefäßen (via Katheter) drosseln. Der minimale Innendurchmesser sollte dabei 4 mm nicht unterschreiten, wenn das Gefäß offen bleiben muß. (Eine Anwendungsmöglichkeit ist das pulmonale Banding bei Kindern mit Links-Rechts-Shunt).

Für einen Verschluß besonders geeignet ist eine Form, die hier als Schnecke bezeichnet wird. In diesem Fall ist die Primärform schneckenförmig aufgewickelt, wobei im Zentrum die Primärform im rechten Winkel von der Schnecke abgebogen ist und in Abstand von der ersten Schnecke parallel zu dieser eine zweite Schnecke ausgebildet ist, die ggf. auch einen kleineren oder größeren Durchmesser aufweisen kann. Die Primärform zwischen den beiden Schneckenelementen kann auch als Spirale gewickelt sein, um der Sekundärform in Richtung der Längsachse eine größere Stabilität zu verleihen. Diese kann bei Herzseptumdefektverschluß den Wanddickenänderungen bei jedem Herzschlag elastisch nachgeben.

Zur Erhöhung der Steifigkeit der Außenwindungen der Schnecke oder der Doppelkonusform kann der zur Herstellung der Primärspirale oder Primärform verwendete Draht statt eines größeren runden Querschnittes auch einen ovalen oder rechteckigen Querschnitt aufweisen.

Da besonders im perinatalen Einsatz der Außendurchmesser des Einführungskatheters möglichst klein sein sollte, und deshalb auch der Spiraldurchmesser der Sekundärform zwangsweise klein bleiben muß, kann das bei größeren zu verschließenden Organwegen zu Stabilitätsproblemen bei der Anordnung und Plazierung führen, mit der Gefahr des unvollständigen Verschlußes oder der Dislozierung. In einem solchen Falle ist es besonders bevorzugt, Doppelkonen als Implantate in einer Tandemanordnung im Organweg zu plazieren. Zur Plazierung werden zwei jeweils als Doppelkonus ausgebildete Sekundärformen hintereinander auf dem Führungsdraht in gestreckter Form angeordnet. Die erste Doppelkonusform mit einem bleibenden Innenlumen wird implantiert und anschließend das zweite Implantat in gestreckter Form soweit vorgeschoben, daß das vordere Ende über den zuerst implantierten Doppelkonus hinausragt und teilweise entrollt. Durch weiteres Zurückziehen des Führungsdrahtes bei feststehendem Abstreifer wird ein Mittelteil des Doppelkonus im Inneren des ersten Doppelkonus in mehr oder weniger gestreckter Form abgelagert und das Ende des zweiten Doppelkonus außerhalb des ersten Doppelkonus entrollt. Die Federkraft der im Innenlumen des ersten Doppelkonus noch gestreckten zweiten Implantats zieht das erste Implantat möglichst eng zusammen und vergrößert den Außendurchmesser der Enden des Doppelkonus und erhöht die Andruckkraft an die Organwand. Gleichzeitig wird das Innenlumen zusätzlich verschlossen. Der minimale Außendurchmesser beim Katheterverschluß beträgt bei dieser Technik nur 1,3 bis 2,3 mm (4 F bis 7 F), so daß diese Implantierungstechnik auch bei Neu- oder Frühgeborenen einsetzbar ist. Erfolgt die Zuführung des kleinsten Implantats in einer normalen Kanüle so beträgt der kleinste Außendurchmesser nur 1 mm und gestattet so relativ gefahrlos bereits den pränatalen Einsatz, z.B. zum Aneurysmaverschluß.

Da beim Verschluß eines Organweges thrombotische Wirkung erwünscht ist, wird die Oberfläche dieser Implantate nicht heparinisiert, sondern im Gegenteil, gerinnungsfördernd ausgebildet. Dies kann durch Belegen der Oberfläche mit Metallpartikeln, Siliconen, Polytetrafluorethylen, medizinisch verträglichen Kautschuklatices oder medizinisch verträglichen, die Blutgerinnung fördernden Polymeren erfolgen.

Zur Austamponierung von Hohlräumen und Gefäßen ist insbesondere eine Sekundärform des Implantats geeignet, die mehrere hintereinanderliegende Schlingen in Form von liegenden Achten aufweist.

Die Erfindung wird nachfolgend unter Bezugnahme auf die Abbildung noch detaillierter beschrieben.

Abbildung 1 zeigt schematisch eine zylindrische Sekundärform des Implantats, die bei kleinem Spiralendurchmesser zum Verschließen eines engen Organweges oder bei größerem Durchmesser als Prothese zum Offenhalten eines Organweges verwendet werden kann.

Abbildung 2 zeigt im Längsschnitt schematisch ein Teilstück des hinteren Endes der Primärform der Implantatspirale von Abbildung 1.

Abbildung 3 zeigt schematisch eine zylindrische Sekundärform der Implantatspirale, deren Primärform sich in der Querschnittsform von der Primärform in Abbildung 1 unterscheidet.

Abbildung 4 zeigt schematisch eine zylindrische Sekundärform des Implantats mit Spiralwindungen unterschiedlichen Durchmessers.

Abbildung 5 zeigt schematisch eine konische Form des Implantats.

Abbildung 6 zeigt schematisch ein Implantat in Form eines Doppelkonus.

Abbildungen 7, 7a und 7b zeigen die Implantatform der sogenannten Doppelschnecke und Doppelrosette von der Seite und von hinten.

Abbildung 8 zeigt schematisch eine bevorzugte Ausführungsform des vorderen Endes eines Einführdrahtes zum Implantieren der erfindungsgemäßen Spiralen.

Abbildung 9 zeigt das vordere Ende eines Einführkatheters im Längsschnitt.

Abbildung 10 zeigt das in Abbildung 9 wiedergegebene Ende eines Einführkatheters im Querschnitt.

Abbildung 11 zeigt in der Folge a bis e schematisch die einzelnen Schritte des Plazierens eines erfindungsgemäßen Implantats in einen Organweg.

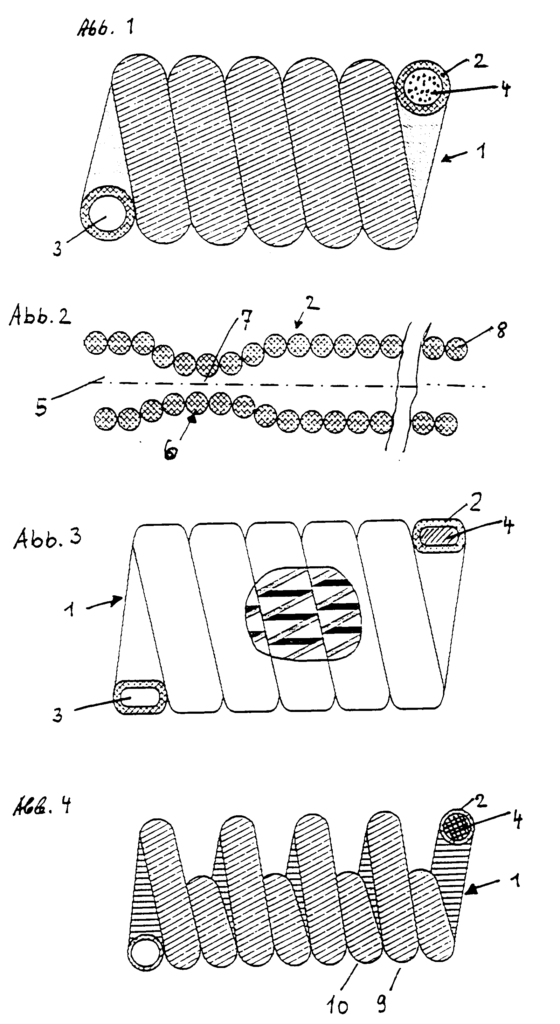

Das in Abbildung 1 gezeigte Implantat hat die Sekundärform 1 einer zylindrischen Wendel oder Spirale, wobei die Wendel 1 selbst aus einer Drahtspirale 2 (Primärform) aus Federmetall besteht. Die Modifizierung des Querschnittes 3 der Primärspirale 2 am hinteren Ende ist in dieser Abbildung nicht gezeigt. Das vordere Ende 4 der Primärspirale 2 ist geschlossen, um sicherzustellen, daß beim Strecken der Sekundärform auf dem Einführungsdraht die Primärspirale 2 nicht so weit auf den Einführungsdraht eingeschoben werden kann, daß dieser über das Ende der Primärspirale 2 hinausragt.

Im Abbildung 2 ist das hintere Ende der Primärform des Implantats von Abbildung 1 in vergrößerter Form wiedergegeben. Der Innendurchmesser der aus Metalldraht mit Federeigenschaften gewickelten Primärspirale 2 ist in geringem Abstand vom hinteren Ende 5 durch Windungen 6 mit geringerem Durchmesser verengt. Wird die Primärspirale 2 auf einen Einführungsdraht aufgeschoben, dessen Außendurchmesser etwas größer ist als der modifizierte Innendurchmesser 7 der Primärspirale 2, ist der für die Replazierbarkeit gewünschte Klemmsitz der gestreckten Sekundärform auf dem Einführungsdrahtes ausgebildet.

Die in Abbildung 3 wiedergegebene zylindrische Spirale 1 ist aus einer Primärform 2 gebildet, deren Querschnitt 3 oval ist. Das vordere Ende 4 der Primärspirale 2 ist verschlossen. Diese Form ist besonders als Prothese für Organwege geeignet, weil gegenüber der in Abbildung 1 gezeigten Form ein etwas größeres freies Innenvolumen der Prothese verbleibt.

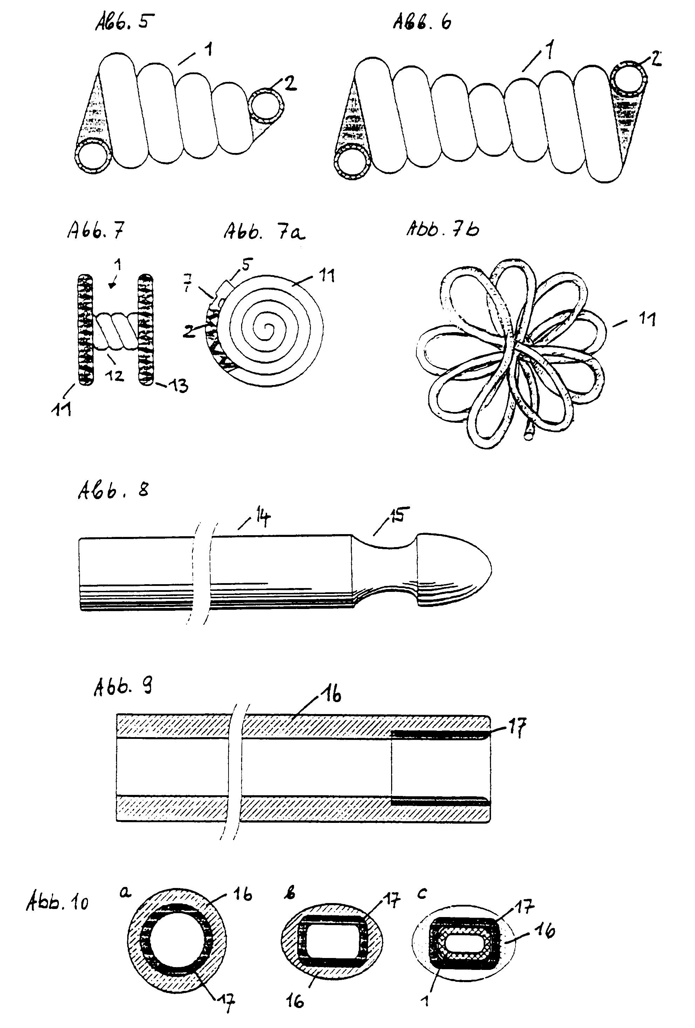

Abbildung 4 zeigt eine zylindrische Form der Sekundärform 1, die aus einer Primärspirale 2 ausgebildet wurde. Das vordere Ende 4 der Primärspirale 2 ist verschlossen. Um das Implantat besonders geeignet für das Verschließen von Organwegen auszubilden, weist die Sekundärform 1 Windungen 9, 10 mit unterschiedlichem Durchmesser auf, die sich jeweils abwechseln. Die Windungen mit größerem Durchmesser 9 des Implantats dienen zum Abstützen des Implantats an der Wand des Organweges und die Windungen 10 mit geringerem Durchmesser verringern das freie Innenvolumen des Implantats und fördern so den Verschluß durch Thrombosierung.

Abbildung 5 zeigt die Ausbildung einer konischen Sekundärform 1 aus einer Primärspirale 2. Um das Plazieren und Wiedereinziehen in den Einführungskatheter einer teilweise ausgerollten, aber noch nicht vom Führungsdraht vollständig abgestreiften Spirale zu erleichtern, wird bei der konischen Ausbildung vorzugsweise die Querschnittsmodifikation in Abstand vom Ende mit geringerem Durchmesser der Sekundärspirale 1 angeordnet. Die Modifikation des Querschnitts kann jedoch auch am Ende der Spirale mit dem größeren Durchmesser angeordnet werden.

Abbildung 6 ist eine besonders bevorzugte Ausführungsform des erfindungsgemäßen spiralförmigen Implantats in Form eines Doppelkonus, wobei die Windungen der Sekundärform 1 mit kleinerem Durchmesser im mittleren Bereich angeordnet sind und die Enden einen größeren Windungsdurchmesser aufweisen. In der Abbildung ist der Außendurchmesser der Sekundärform 1 an beiden Enden gleich. Grundsätzlich ist es aber auch möglich, den Doppelkonus an beiden Enden mit unterschiedlichem Durchmesser der Sekundärform auszubilden.

Abbildung 7 zeigt die hier als Doppelschnecke bezeichnete Form des erfindungsgemäßen Implantats. Die Sekundärform 1 besteht aus zwei parallelen, einen Abstand voneinander aufweisenden, schneckenförmig aufgewickelten Elementen 11 und 13, die miteinander durch ein zylindrisch gewickeltes Zwischenstück 12 verbunden sind, das in Richtung der Längsachse der Doppelstücke verläuft, wobei sich die Elemente 11 und 13 im rechten Winkel zur Längsachse erstrecken. Grundsätzlich ist es aber auch möglich, die Doppelschnecke an beiden Enden mit unterschiedlichen Durchmessern auszubilden.

Die Abbildung 7a zeigt eines der schneckenförmig aufgerollten Elemente 11 mit schematisch angedeuteter Primärform, deren Durchmesser in geringem Abstand vom hinteren Ende 5 im Abschnitt 7 modifiziert, d.h. verengt ausgebildet ist.

Aus Abbildung 7b ist ersichtlich, daß die schneckenförmig aufgerollten Elemente 11 auch als Rosette ausgebildet sein können um anstelle einer Doppelschnecke eine Doppelrosette auszubilden.

Selbstverständlich können alle aus Spiralen (Primärform) gebildeten erfindungsgemäßen bevorzugten Sekundärformen auch aus elastischen Kunststoffrohren gebildet werden.

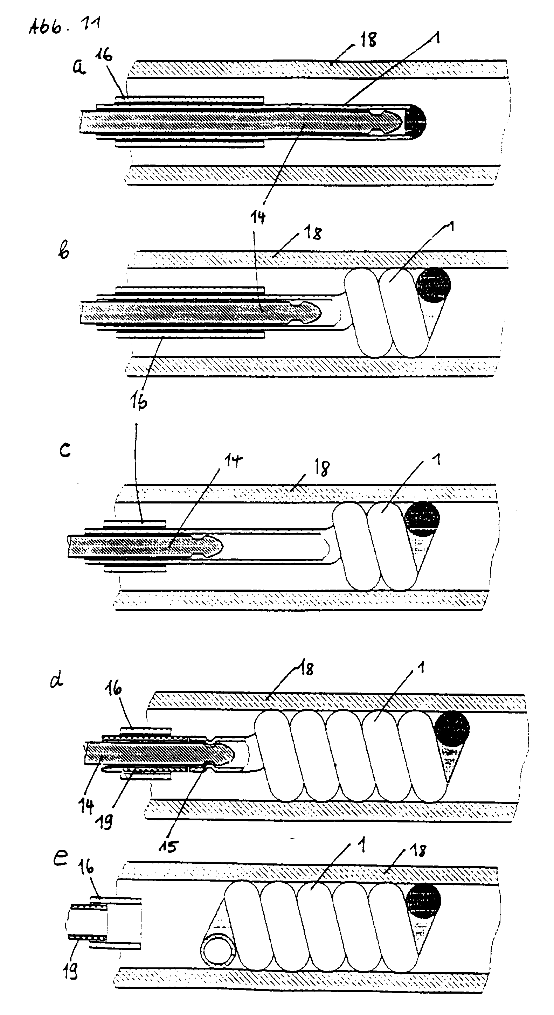

Um den Klemmsitz der erfindungsgemäßen Implantate auf dem Einführungsdraht in einer bestimmten Stellung kurz vor dem endgültigen Ablösen zu unterstützen, kann ein Führungsdraht verwendet werden, dessen vorderes Ende in Abbildung 8 schematisch wiedergegeben ist. Um das Aufschieben der Primärform des Implantats auf den Einführungsdraht 14 zu erleichtern, ist dessen vorderes Ende vorzugsweise konisch abgerundet und in Abstand von der vorderen Spitze eine Ringnut 15 ausgebildet, in die der Abschnitt mit modifiziertem Querschnitt der Primärform des erfindungsgemäßen Implantats eingreift, so daß der Klemmsitz an dieser Stelle besonders ausgeprägt ist und die Maximal kraft zum Überwinden des Klemmsitzes unter Abstreifen des Implantats vom Führungsdraht durch die Kraft zum Lösen des Eingriffs der modifizierten Windungen der Primärspirale oder des modifizierten Querschnittes eines Primärrohres mit der Ringnut 15 bestimmt wird.

Abbildung 9 zeigt im Längsschnitt das vordere Ende eines zum Einführen der erfindungsgemäßen Implantate in Organwege geeigneten Katheters 16, in dessen Spitze vorzugsweise eine Buchse 17 aus Metall oder einem harten, besonders gleitfähigen Polymermaterial fest eingefügt ist. Um das Wiedereinziehen von bereits teilweise gebildeten Sekundärspiralen zum Zwecke der Replazierung in den Katheter zu erleichtern, ist die nach außen gewandte Stirnseite der Buchse nach innen abgerundet. Diese Verstärkungsbuchse streckt den bereits vom führungsdraht abgeschobenen Teil des Implantats so weit, daß er nach Einziehen in den Katheter aus dem Organweg zusammen mit dem Katheter wieder entfernt werden kann, um eine Replazierung bei einem neuen Einführzyklus zu ermöglichen. Dies kann sich insbesondere dann als erforderlich erweisen, wenn sich bei der teilweisen Ausbildung der Sekundärform im Organweg herausstellt, daß eine ungeeignete Implantatform gewählt wurde oder die Ausbildung der Sekundärform nicht in korrekter Lage im Organweg erfolgte.

In Abbildung 10 wird die vorzugsweise verstärkte Katheterspitze im Querschnitt gezeigt.

Abbildung 10a zeigt einen runden Katheter 16 mit einer eingesetzten zylindrischen Buchse 17.

In Abbildung 10b ist ein oval er Katheter 16 mit einer rechteckigen Buchse 17 wiedergegeben und in Abbildung 10c ein ovaler Katheter 16 mit einer rechteckigen Buchse 17, in die ein Implantat 1 zurückgezogen wurde.

Diese rechteckige Form einer Buchse 17 ist besonders dann bevorzugt, wenn die Primärform des Implantats 1 keinen runden, sondern ovalen Querschnitt aufweist.

Abbildung 11 zeigt schematisch das Plazieren des Implantats 1 in einem Organweg 18, beispielsweise einer Blutbahn. Ein Katheter 16 wird innerhalb des Organweges 18 in die Nähe der Stelle geschoben, in der das Implantat 1 plaziert werden soll. Im Katheterinnenlumen befindet sich das Implantat 1 in gestrecktem Zustand in der sogenannten Primärform auf dem Einführdraht 14. Von dem außerhalb des Körpers befindlichen distalen Ende des Katheters aus wird der Führungsdraht 14 mit dem darauf in gestreckter Form befindlichen Implantat 1 in den Organweg 18 an die zu plazierende Stelle vorgeschoben. In Schritt B wird durch Zurückziehen des Führungsdrahtes 14 oder Vorschieben des Abstreifers 19 aus der Primärform des Implantates 1 aufgrund der Federwirkung die Sekundärform des Implantats im Organweg 18 ausgebildet. In Schritt C wird durch Zurückziehen des Einführungskatheters 16 und weiteres Zurückziehen des Führungsdrahtes 14 oder Vorschieben des Abstreifers 19 die Sekundärform des Implantats 1 weiterentwickelt und in Schritt D die Stellung erreicht, in der der Führungsdraht 14 im letzten Stück der Primärform des Implantats 1 enthalten ist und noch Klemmsitz aufgrund des modifizierten Querschnittes 7 der Primärform des Implantats 1 auf dem Führungsdraht 14 vorhanden ist. Bei der in Schritt D wiedergegebenen Ausführungsform des Führungsdrahtes weist dieser eine ringförmige Nut 15 auf, in die die Wandung des modifizierten Querschnittes der Primärform, beispielsweise ein oder mehrere Spiralen eines Drahtes 6 mit geringeren oder zumindest in einer Richtung verringerten Querschnittes eingreifen. In dieser Stellung ist nach Kontrolle der richtigen Plazierung des Implantats 1 im Organweg 18 letztmalig ein Zurückziehen der entrollten Sekundärform des Implantats 1 in den Katheter 16 durch Zurückziehen des Führungsdrahtes möglich. Dadurch, daß die Kraft zum Überwinden des Klemmsitzes ein Mehrfaches der Kraft beträgt, die erforderlich ist, die ausgebildete Sekundärform des Implantats 1 wieder zu strecken und in den Katheter 16 zurückzuziehen, ist die Replazierbarkeit der erfindungsgemäßen Implantate bei inkorrekter Anordnung oder ungeeigneter Wahl der Form möglich. Auf dem Führungsdraht 14 ist in axialer Richtung hinter der Primärform des Implantats 1 ein Abstreifer 19 angeordnet, dessen hinteres Ende bis zum distalen Ende des Katheters reicht. Der Abstreifer kann ein Rohr oder aus Elastizitätsgründen auch eine Spirale sein, deren Außendurchmesser mit dem Außendurchmesser der Primärform des Implantates am hinteren Ende übereinstimmt. Durch Vorschieben des Abstreifers 19 im Katheter 16 oder, bei feststehendem Abstreifer 19, Zurückziehen des Führungsdrahtes 14 wird der Klemmsitz des Implantats 1 auf dem Führungsdraht 14 gelöst und das Ende des Führungsdrahtes aus der Form herausgezogen bzw. die Primärform vom Führungsdrahtende abgestreift. Das Implantat 1 nimmt dann die vorgegebene Form im Organweg 18 ein. Katheter 16 und Abstreifer 19 werden aus dem Organweg zurückgezogen.

Im Falle einer Tandemanordnung von Implantaten werden zwei Primärformen in Tandemanordnung auf einem Führungsdraht gestreckt, vorzugsweise Implantate mit der Doppelkonusform. Zunächst wird das erste Implantat im Organweg plaziert und anschließend im Innenlumen des Implantats das zweite Implantat entrollt und plaziert. Im Falle von doppelkonischen Implantaten ist es möglich, diese so anzuordnen, daß der mittlere Teil des zweiten Implantats zumindest noch teilweise in gestreckter Form verbleibt und so eine Zugwirkung auf die Enden des doppelkonischen ersten Implantats ausübt.

Der besondere Vorteil der erfindungsgemäßen Ausbildung von spiralförmigen Implantaten besteht darin, daß das Implantat auch im implantierten Zustand noch manipuliert werden kann, solange es noch nicht vollständig vom Führungsdraht abgekoppelt ist. Die dadurch gegebene Replazierfähigkeit stellt die entscheidende Verbesserung gegenüber der herkömmlichen Technik dar. Nach Thrombosierung im Bereich des Implantates ist ein dauerhafter Verschluß des Organweges, beispielsweise von Blutgefäßen gegeben. Werden resorbierbare Implantatmaterialien mit Federwirkung verwendet, sind auch langfristig keine unerwünschten Nebenwirkungen zu erwarten. Die Kontrolle der exakten Plazierung kann radiologisch oder mittels Ultraschall erfolgen.

## Patentansprüche

1. Implantat für Organwege, das aus einer Primärspirale (2) aus Metall oder einem Primärrohr (2) aus elastischem Kunststoff gebildet wird, wobei das vordere Ende (4) der Primärspirale (2) oder des Primärrohres (2) geschlossen ist und der hintere Bereich als Klemmsitz für einen Führungsdraht ausgebildet ist und das Implantat eine Sekundärform (1) vergrößerten Außendurchmessers aufweist, die durch Aufschieben der Primärspirale (2) oder des Primärrohres (2) auf den Führungsdraht (14) gestreckt werden kann und sich bei Herausziehen des Fürhungsdrahtes (14) oder Abschieben vom Führungsdraht (14) durch im Material vorhandene Rückstellkräfte zurückbildet,
**dadurch gekennzeichnet**,
daß in Abstand von 0,5 mm bis 2 mm vom hinteren Ende der Primärspirale (2) oder des Primärrohres (2), deren/ dessen Querschnitt (7) auf einer Strecke von 0,01 mm bis 10 mm durch Verringerung des Innendurchmessers in mindestens einer radialen Richtung modifiziert ist.

2. Implantat nach Anspruch 1,
**dadurch gekennzeichnet**,
daß der Drahtdurchmesser der Primärspirale (2) 0,06 bis 0,6 mm und der Durchmesser der Primärspirale (2) von 0,2 bis 3 mm beträgt.

3. Implantat nach Anspruch 1,
**dadurch gekennzeichnet**,
daß die Wandstärke des Primärrohres (2) aus Kunststoff von 0,08 mm bis 0,8 mm und der Durchmesser von 0,3 bis 3 mm beträgt.

4. Implantat nach Anspruch 1 oder 2,
**dadurch gekennzeichnet**,
daß der Querschnitt mindestens einer bis mehrerer Windungen (6) der Primärspirale (2) durch Verringerung des Durchmessers der Primärspirale (2) oder Ausbilden eines ovalen Querschnittes mit gegenüber dem ovalen Durchmesser reduziertem Durchmesser der Primärspirale (2) modifiziert ist.

5. Implantat nach Anspruch 1 oder 3,
**dadurch gekennzeichnet**,
daß der Querschnitt des Primärrohres (2) durch Verringern des Durchmessers oder Ausbilden eines ovalen Querschnittes mit in einer Richtung gegenüber dem normalen Durchmesser verringerten Durchmesser modifiziert ist.

6. Implantat nach Anspruch 1,
**dadurch gekennzeichnet**,
daß die Primärspirale (2) oder das Primärrohr (2) 10 bis 500 mm lang ist.

7. Implantat nach Anspruch 1,
**dadurch gekennzeichnet**,
daß die Sekundärform (1) durch Verwindung von Primärspirale (2) oder Primärrohr (2) die Form eines Zylinders, eines Konus, ein Doppelkonus mit größeren Durchmessern an den Enden, eines Zylinders, bei dem sich Windungen (9, 10) der Sekundärform (1) mit unterschiedlichen Durchmessern abwechseln, eine durch einen zylindrischen Abschnitt (12) verbundene Doppelschnecke (11, 13), Doppelrosette oder die Form von mehreren liegenden Achten hat.

8. Implantat nach Anspruch 1,
**dadurch gekennzeichnet**,
daß die Primärspirale (2) aus einem Draht mit runden, ovalen oder rechteckigem Querschnitt gewickelt ist.

9. Implantat nach Anspruch 7,
**dadurch gekennzeichnet,**
daß die inneren Windungen des Doppelkonus oder der Doppelschnecke oder der Doppelrosette aus einer Primärspirale (2) mit rundem Drahtquerschnitt bestehen und die äußeren Windungen aus einer Primärspirale (2) mit einem vergrößerten runden, ovalen oder rechteckigen Drahtquerschnitt bestehen.

10. Kombination einer Vorrichtung zur Einführung eines Implantats in einen Organweg mit Einführkatheter (16), Abstreifer (19) und Führungsdraht (14) für das Implantat mit einem Implantat nach Ansprüchen 1-9,
**dadurch gekennzeichnet**,
daß die zum Verschieben der Primärspirale (2) oder des Primärrohres (2) und zum Überwinden der Klemmkraft mit dem als Einführhilfe dienenden Führungsdraht (14) erforderliche Kraft 0.5 N bis 10 N beträgt.

11. Kombination nach Anspruch 10,
**dadurch gekennzeichnet**,
daß der Führungsdraht (14) am vorderen oder in der Nähe des Ende(s) entweder mit einer Ringnut (15) oder einem umlaufenden Wulst versehen ist.

## Claims

1. Implant for organ pathways, which is formed from a primary spiral (2) made of metal or a primary tube (2) made of elastic synthetic material, where the anterior end (4) of the primary spiral (2) or of the primary tube (2) is closed and the posterior region is formed as a clamping seat for a guide wire, and the implant has a secondary shape (1) of increased external diameter, which shape can be extended by sliding the primary spiral (2) or the primary tube (2) onto the guide wire (14) and reforms on pulling out the guide wire (14) or on pushing off from the guide wire (14) as a result of restitutory forces present in the material, characterized in that, at a distance of 0.5 mm to 2 mm from the posterior end of the primary spiral (2) or of the primary tube (2) its cross-section (7) is modified for a distance of 0.01 mm to 10 mm by constriction of the internal diameter in at least one radial direction.

2. Implant according to claim 1, characterized in that the wire diameter of the primary spiral (2) is 0.06 to 0.6 mm and the diameter of the primary spiral (2) is from 0.2 to 3 mm.

3. Implant according to claim 1, characterized in that the wall thickness of the primary tube (2) made of synthetic material is from 0.08 mm to 0.8 mm and the diameter is from 0.3 to 3 mm.

4. Implant according to claim 1 or 2, characterized in that the cross-section of at least one to several turns (6) of the primary spiral (2) is modified by constricting the diameter of the primary spiral (2) or by forming an oval cross-section having a reduced diameter of the primary spiral (2) as compared with the oval diameter.

5. Implant according to claim 1 or 2, characterized in that the cross-section of the primary tube (2) is modified by constricting the diameter, or by forming an oval cross-section having a reduced diameter in one direction as compared with the normal diameter.

6. Implant according to claim 1, characterized in that the primary spiral (2) or the primary tube (2) is 10 to 500 mm long.

7. Implant according to claim 1, characterized in that the secondary shape (1), by twisting a primary spiral (2) or primary tube (2), has the form of a cylinder, a cone, a double cone with larger diameters at the ends, a cylinder in which turns (9, 10) of the secondary shape (1) having different diameters alternate, a double helix (11, 13) which is connected by a cylindrical section (12), a double rosette, or the form of several horizontal figures of eight.

8. Implant according to claim 1, characterized in that the primary spiral (2) is wound from a wire having a round, oval or rectangular cross-section.

9. Implant according to claim 7, characterized in that the inner turns of the double cone or of the double helix or of the double rosette are composed of a primary spiral (2) having a round wire cross-section, and the outer turns are composed of a primary spiral (2) having an enlarged round, oval or rectangular wire cross-section.

10. Device for inserting an implant according to one of claims 1 to 9 into an organ pathway, having an insertion catheter (16), a stripping element (19) and a guide wire (14) for the implant, characterized in that the force required to displace the primary spiral (2) or the primary tube (2) and to overcome the clamping force with the guide wire (14) serving as an insertion aid is 0.5 N to 10 N.

11. Device according to claim 10, characterized in that the guide wire (14) is provided, at the anterior end or in the vicinity of this end, either with an annular groove (15) or with a circumferential beading.

## Revendications

1. Implant pour conduits du corps qui est constitué d'une hélice primaire (2) en métal ou d'un tube primaire (2) en matière plastique élastique, l'extrémité avant (4) de l'hélice primaire (2) ou du tube primaire (2) étant fermée et la partie arrière se présentant sous forme d'un siège de blocage pour un fil de guidage et l'implant présentant une forme secondaire (1) de diamètre extérieur agrandi qui peut être étendue par enfilement de l'hélice primaire (2) ou du tube primaire (2) sur le fil de guidage (14) et se rétablit, lors de l'extraction du fil de guidage (14) ou du dégagement du fil de guidage (14), grâce à des forces de rappel présentes dans le matériau, caractérisé en ce qu'à une distance de 0,5 mm à 2 mm de l'extrémité arrière de la spirale primaire (2) ou du tube primaire (2), sa section (7) est modifiée sur une longueur de 0,01 mm à 10 mm par rétrécissement du diamètre Intérieur, au moins dans un sens radial.

2. Implant suivant la revendication 1, caractérisé en ce que le diamètre de fil de l'hélice primaire (2) est de 0,06 à 0,6 mm et le diamètre de l'hélice primaire (2) est de 0,2 à 3 mm.

3. Implant suivant la revendication 1, caractérisé en ce que l'épaisseur de paroi du tube primaire (2) en matière plastique est de 0,08 mm à 0,8 mm et le diamètre de 0,3 à 3 mm.

4. Implant suivant la revendication 1 ou 2, caractérisé en ce que la section d'au moins une à plusieurs spires (6) de l'hélice primaire (2) est modifiée par rétrécissement du diamètre de l'hélice primaire (2) ou formation d'une section ovale ayant le diamètre de l'hélice primaire (2) réduit par rapport au diamètre oval.

5. Implant suivant la revendication 1 ou 3, caractérisé en ce que la section du tube primaire (2) est modifiée par rétrécissement du diamètre ou formation d'une section ovale ayant un diamètre réduit, selon un axe, par rapport au diamètre normal.

6. Implant suivant la revendication 1, caractérisé en ce que l'hélice primaire (2) ou le tube primaire (2) a une longueur de 10 à 500 mm.

7. Implant suivant la revendication 1, caractérisé en ce que la forme secondaire (1) a, par gauchissement de l'hélice primaire (2) ou du tube primaire (2), la forme d'un cylindre, d'un cône, d'un double cône à diamètres plus grands aux extrémités, d'un cylindre dans lequel alternent des spires (9, 10) de la forme secondaire (1) de diamètres différents, d'une double spirale (11, 13) reliée par un tronçon cylindrique (12), d'une double rosace ou la forme de plusieurs huits couchés.

8. Implant suivant la revendication 1, caractérisé en ce que l'hélice primaire (2) est enroulée à partir d'un fil de section ronde, ovale ou rectangulaire.

9. Implant suivant la revendication 7, caractérisé en ce que les spires intérieures du double cône ou de la double spirale ou de la double rosace consistent en une hélice primaire (2) à section de fil ronde et que les spires extérieures consistent en une hélice primaire (2) à section de fil ronde, ovale ou rectangulaire agrandie.

10. Combinaison d'un dispositif pour l'introduction d'un implant dans un conduit du corps, avec un cathéter d'introduction (16), un racle (19) et un fil de guidage (14) pour l'implant, avec un implant suivant les revendications 1 à 9, caractérisée en ce que la force nécessaire pour déplacer l'hélice primaire (2) ou le tube primaire (2) et pour vaincre la force de serrage avec le fil de guidage (14) servant d'aide d'introduction est de 0,5 N à 10 N.

11. Combinaison suivant la revendication 10, caractérisée en ce que le fil de guidage (14) est pourvu, à l'extrémité avant ou dans le voisinage de l'extrémité ou des extrémités, soit d'une rainure annulaire (15), soit d'un rebord périphérique.
